# EUROPEAN PATENT SPECIFICATION

(11) **EP 4 382 154 B1**
(45) Date of publication and mention of the grant of the patent: **26.11.2025**
(21) Application number: 22211525.5
(22) Date of filing: 05.12.2022
(51) Int. Cl.: A61M 15/00, A61M 11/02, A61M 11/00

(54) **DEVICE FOR ADMINISTERING A PREDEFINED QUANTITY OF A COMPOUND TO A PATIENT**
VORRICHTUNG ZUR VERABREICHUNG EINER VORBESTIMMTEN MENGE EINER VERBINDUNG AN EINEN PATIENTEN
DISPOSITIF POUR ADMINISTRER UNE QUANTITÉ PRÉDÉFINIE D'UN COMPOSÉ À UN PATIENT

(43) Date of publication of application: 12.06.2024
(73) Proprietor: Uimei Srls, 24060 Tavernola Bergamasca (BG) (IT)
(72) Inventor: FORESTI, Ruben, 43125 PARMA (IT)
(74) Representative: Dondi, Silvia

(56) References cited:
- EP-A1- 1 731 186
- WO-A1-2004/060458
- WO-A1-2007/015665
- WO-A2-94/11044
- FR-A1- 2 701 653
- US-A- 5 383 850
- US-A- 5 388 572

## Description

The present invention relates to a device for administering a predefined quantity of a compound to a patient.

The invention has application in the administration of volumes of substances (e.g. drugs) in microlitres to patients orally.

The administration by inhalation of substances in gas, liquid or powder form is having exponential growth in preclinical and clinical research, thanks to the enormous development of nano materials for nanomedicine and nanotoxicology studies.

Administration by inhalation is also becoming a sought-after alternative for particular drugs that are less effective and/or reach their target with greater difficulty or more slowly if administered intravenously.

Various types of metered-dose inhalers, i.e. pressurised devices used to administer a pharmaceutical product in the respiratory tract by inhalation, are already known.

Such metered-dose inhalers, indicated in the sector by the acronym MDI, comprise a reservoir containing a pharmaceutical formulation in suspension or in solution in a liquid propellent and a metering valve inserted in the reservoir. Thanks to manual actuation of the dosing valve it is possible to dispense a dose of a product by means of a propellent that acts as a carrier of the pharmaceutical formulation.

The main drawback present in the metered-dose inhalers of the known art lies in the fact that the device contains the compound (e.g. drug) mixed with the propellant or only powdered drug without any propellant.

Another drawback is that the patient must independently manage the therapy prescribed by the doctor.

Document WO 94/11044 A2 discloses a device according to the preamble of claim 1.

In this context, the technical task underpinning the present invention is to propose a device for administering a predefined quantity of a compound to a patient which obviates the drawbacks of the prior art as cited above.

In particular, the object of the present invention is to propose a device for administering a predefined quantity of a compound to a patient, in which the compound is not contained inside the device.

Another object of the present invention is to provide a device for administering a predefined quantity of a compound to a patient, which is easy to use and safe.

The stated technical task and the specified objects are substantially achieved by a device for administering a predefined quantity of a compound to a patient according to the appended set of claims.

Further features and advantages of the present invention will become more apparent from the illustrative and thus non-limiting description of a preferred but not exclusive embodiment of a device for administering a predefined quantity of a compound to a patient, as illustrated in the appended drawings, in which:

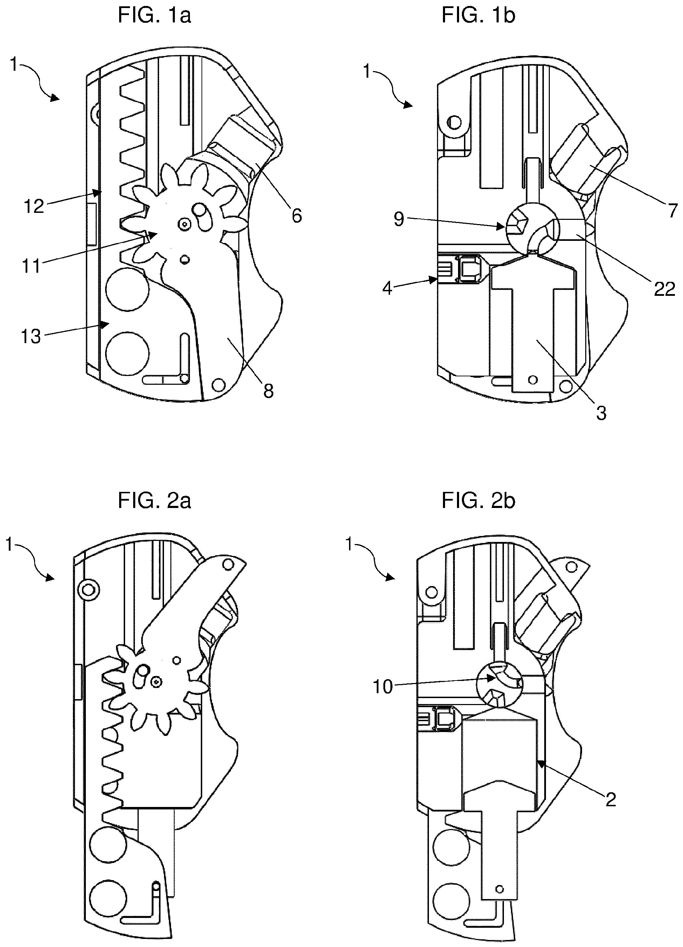

- figures 1a and 1b illustrate a device for administering a predefined quantity of a compound to a patient, not according to the present invention, in a condition of non-use in a side view and a sectional side view, respectively;
- figures 2a and 2b respectively illustrate a side view and a sectional side view of the device of figures 1a and 1b, in a loading condition;

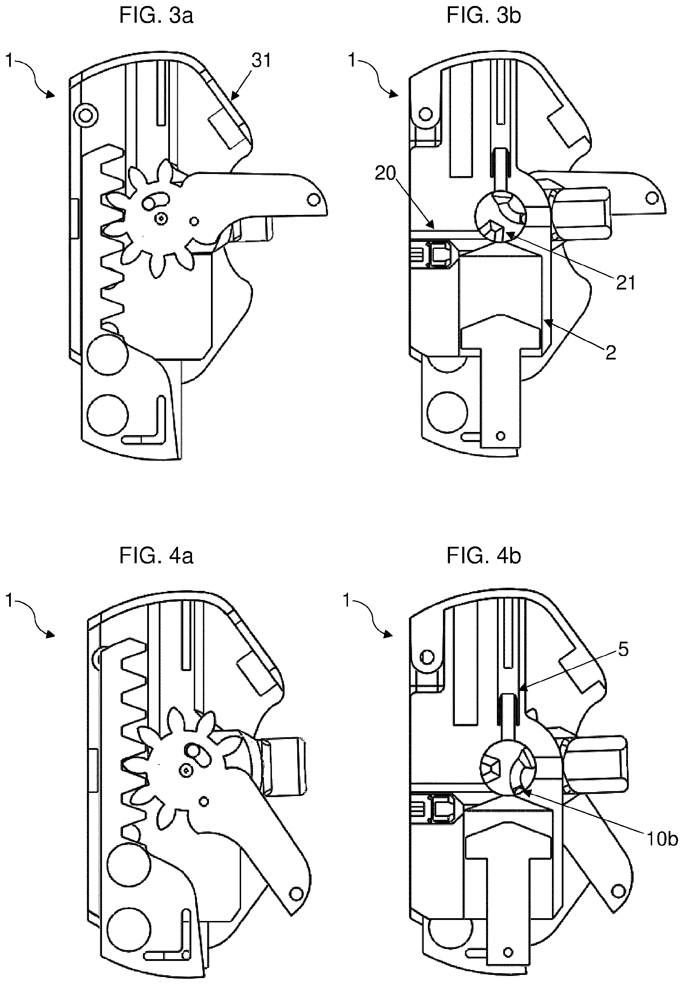

- figures 3a and 3b respectively illustrate a side view and a sectional side view of the device of figures 1a and 1b, with the mouthpiece in operating position;
- figures 4a and 4b respectively illustrate a side view and a sectional side view of the device of figures 1a and 1b, in a third intermediate position of the arm;

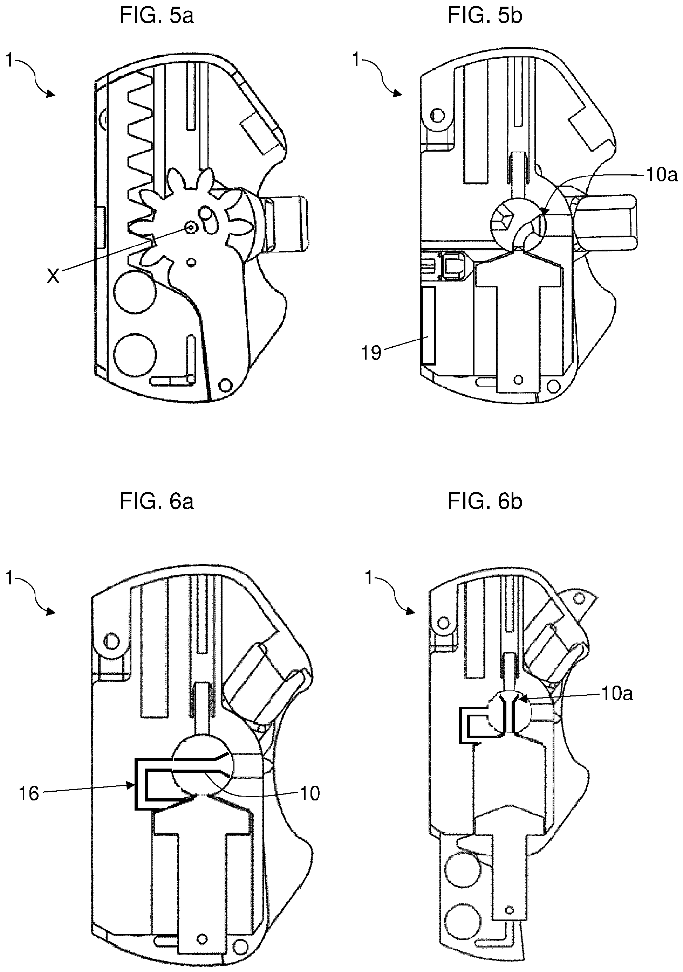

- figures 5a and 5b respectively illustrate a side view and a sectional side view of the device of figures 1a and 1b, in an administration condition;

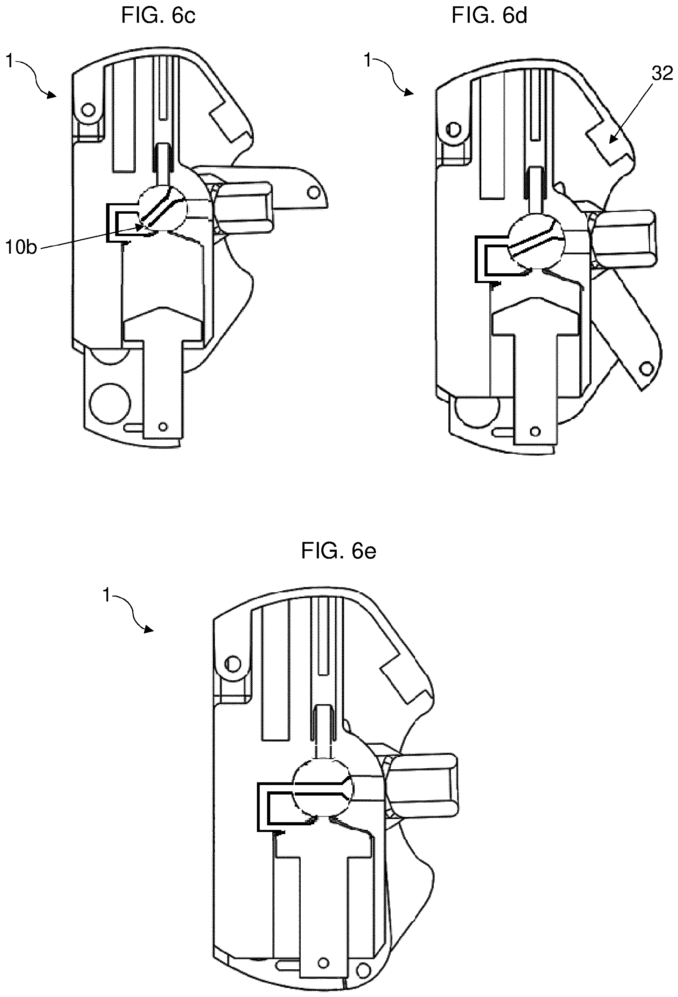

- figures 6a-6e illustrate a sectional side view of a device for administering a predefined quantity of a compound to a patient, according to the present invention, respectively under the conditions illustrated for the in figures 1b, 2b, 3b, 4b, 5b;

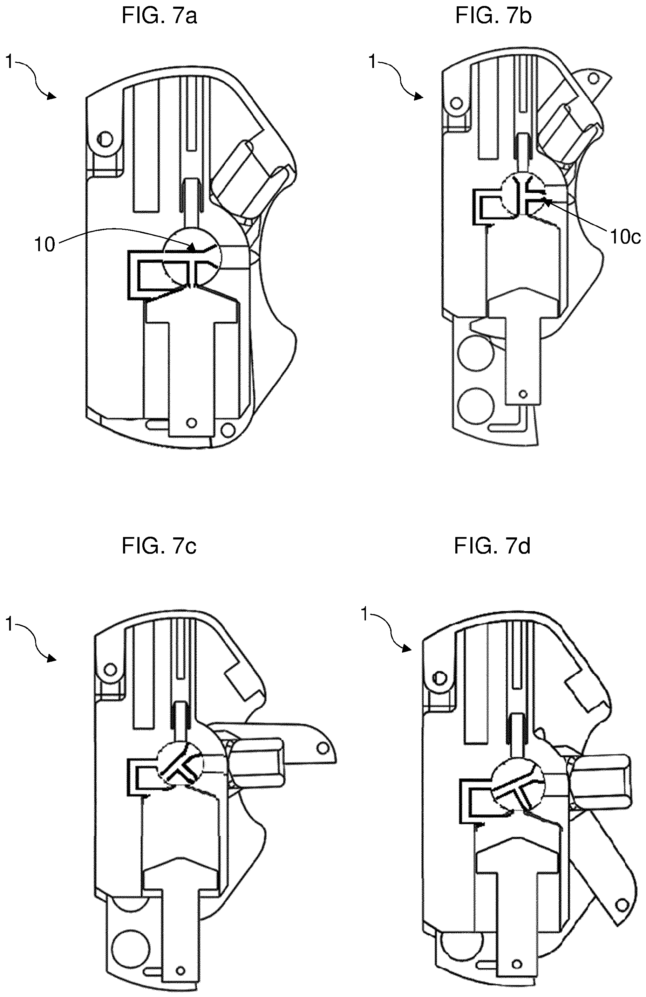

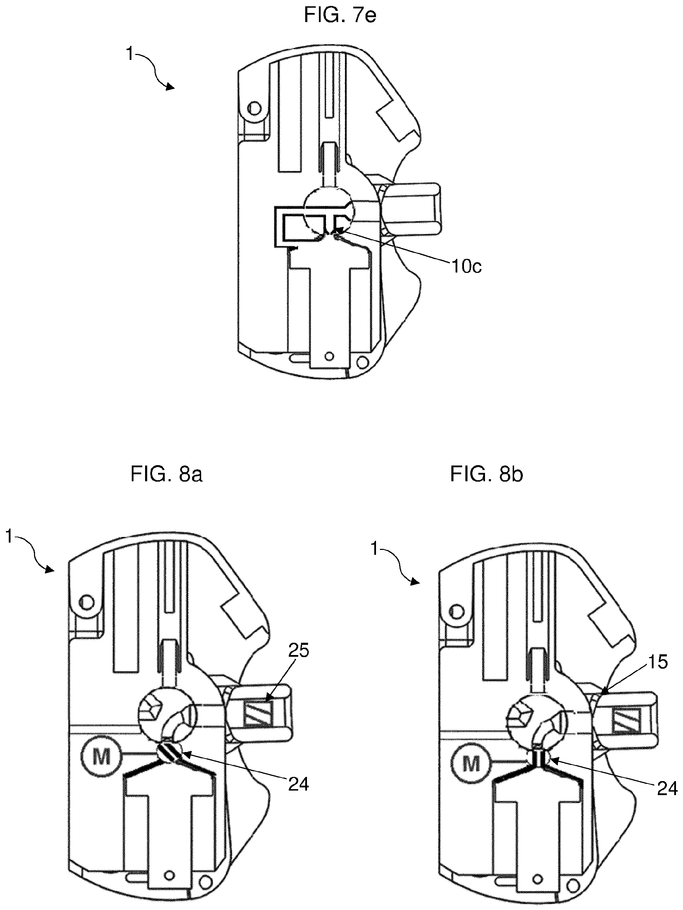

- figures 7a-7e illustrate a sectional side view of an alternative embodiment (three-way) of the dosing conduit of the device, respectively in the conditions illustrated in figures 6a-6e;
- figures 8a and 8b illustrate an alternative embodiment of the device in which a tap is interposed between the dosing body and the reservoir, in two sectional side views with the tap closed and open, respectively;

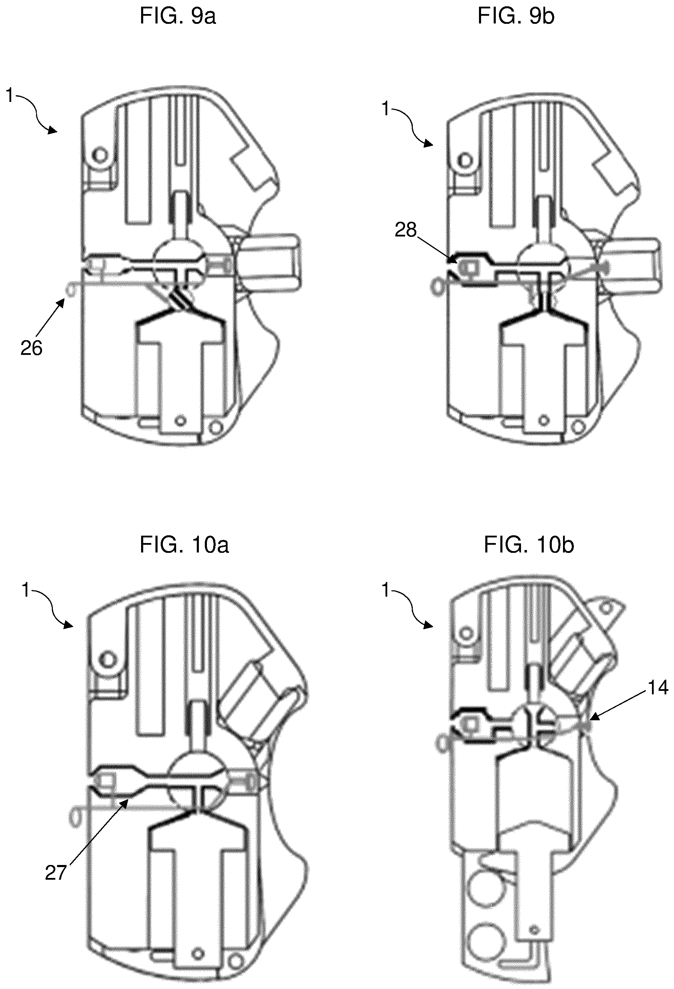

- figures 9a and 9b illustrate an additional embodiment of the device of figures 8a and 8b, in which the communication between the reservoir and the channel occurs by means of a tap and a shutter;

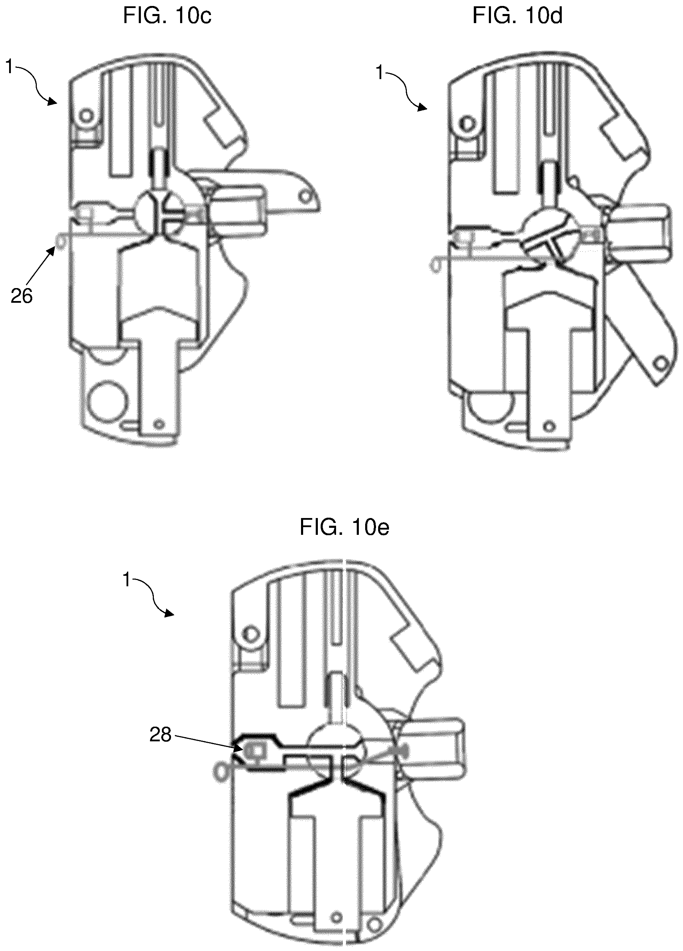

- figures 10a-10e illustrate the device, not according to the present invention, in which the triggering of the administration of the compound to the patient occurs by sucking together with pressing a button, respectively in the conditions illustrated in figures 1b, 2b, 3b, 4b, 5b;

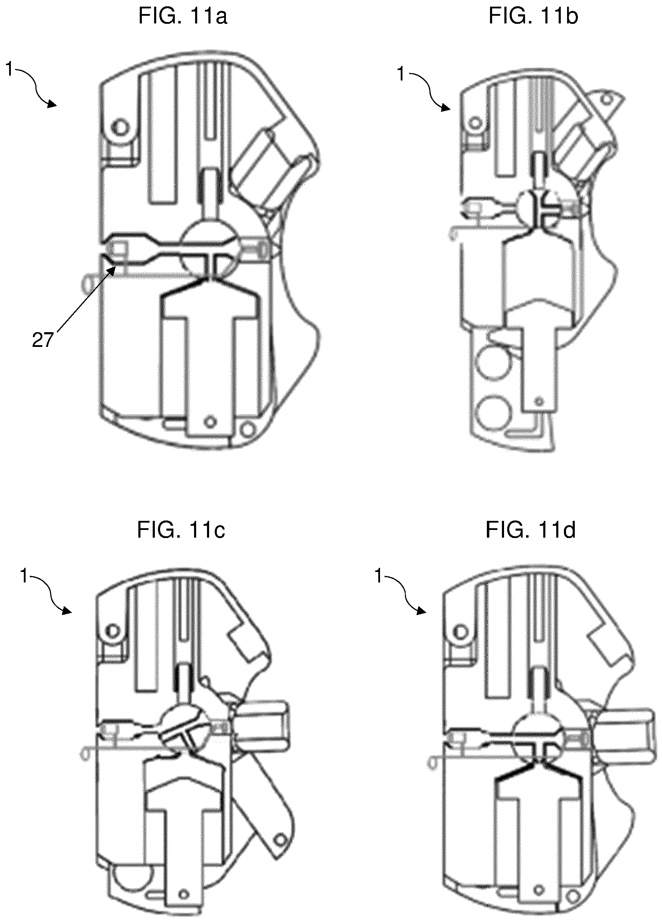

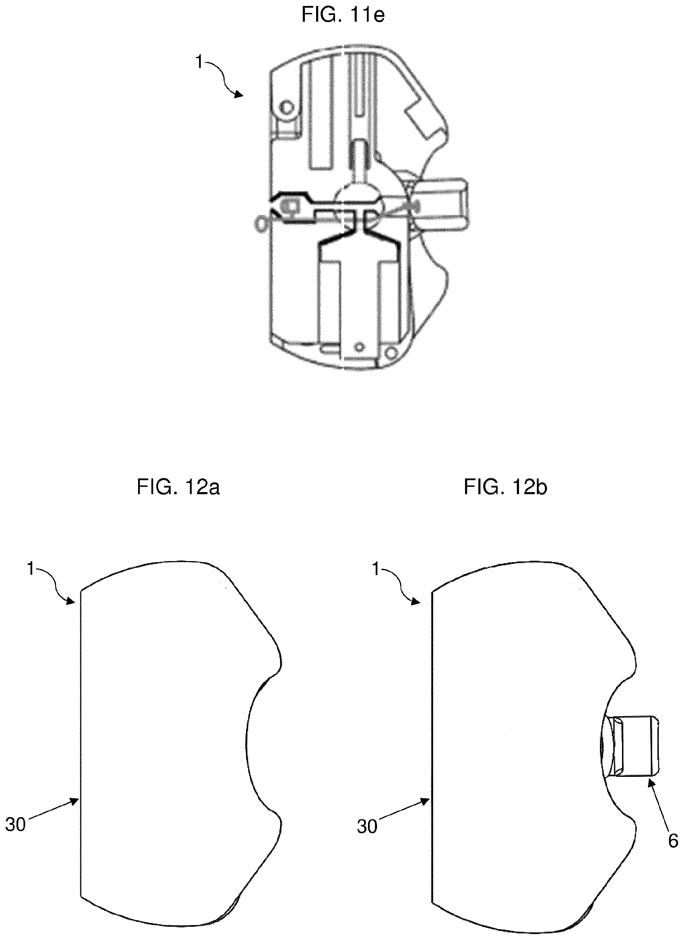

- figures 11a-11e illustrate the according to the present invention, in which the triggering of the administration of the compound to the patient occurs by sucking together with pressing a button, respectively in the conditions illustrated in figures 6a-6e;
- figures 12a and 12b illustrate an outer casing of the device for administering a predefined quantity of a compound to a patient, according to the present invention, respectively in a resting and operating position of the mouthpiece;
- figure 13 illustrates a loading drum of the compound, in perspective view.

With reference to the figures, the number 1 indicates a device for administering a predefined quantity of a compound to a patient. For example, the compound may take on different states: liquid, gas, powder or hydrogel.

In accordance with an aspect of the invention, the compound is a drug.

The device 1 comprises a reservoir 2 of predefined volume. In particular, as a function of needs, it is possible to choose reservoirs of different volume, and they can be connected in series or in parallel.

The device 1 comprises a plunger or piston 3 slidably mounted inside the reservoir 2. Preferably, the device 1 comprises a hollow cylindrical body constituting the reservoir 2 for the gaseous mixture. The plunger 3 is slidably mounted inside the hollow cylindrical body 2.

The device 1 comprises at least one compartment or housing 5 receiving the predefined quantity of the compound.

The device 1 comprises a mouthpiece 6 which the patient receives in the mouth to take the predefined quantity of the compound orally. The mouthpiece 6 is configurable in at least one resting position, in which it is not accessible to a mouth of the patient, and an operating position, in which it is accessible to the mouth of the patient. The mouthpiece 6 comprises an internal channel 7 for expelling the compound.

The device 1 comprises a dosing body 9 in turn comprising one or more dosing conduits 10 therein. In this context, the dosing body 9 is referred to as the "lung".

In particular, the lung 9 is a calibrated lung.

The device 1 further comprises an actuation means 8 of the lung 9, configured to rotate the lung 9 and move it between different positions.

In accordance with an embodiment, the actuation means 8 comprises at least one arm 8 which is rotatable about an axis X between a first position and a second position.

In the embodiment illustrated herein, the dosing body 9 comprises a dosing conduit 10 of the compound. The dosing body 9 is connected to the arm 8 so as to rotate together therewith.

In particular, the dosing body 9 can rotate between:
- a loading position, in which the dosing conduit 10 is in fluid communication with the compartment 5 to receive the predefined quantity of the compound, and
- an administration or dispensing position, in which the dosing conduit 10 is in fluid communication with the reservoir 2 on one side, and with the channel 7 of the mouthpiece 6 (configured in the operating position) on the other.

The loading position of the dosing body 9 corresponds to the first position of the arm 8. The administration position of the dosing body 9 corresponds to the second position of the arm 8.

Preferably, in the first position and in the second position the arm 8 is arranged on opposite sides with respect to the dosing body 9. Preferably, in the first position the arm 8 extends upwards away from the axis X. Preferably, in the second position the arm 8 extends downwards away from the axis X.

The plunger 3 is operatively connected to the actuation means 8 so as to be movable inside the reservoir 2 when the dosing body 9 rotates between the positions. In particular, the plunger 3 is slidable between a first position and a second position. In the first position it is located near the dosing body 9, in the second position it is located distanced from the dosing body 9. That is, in the first position the plunger 3 is located with respect to the dosing body 9 at a shorter distance with respect to the second position.

When the dosing body 9 moves from the loading position to the administration position, the plunger 3 moves from the second position to the first position, i.e. approaches the dosing body 9. In particular, the plunger 3 is in the first position (minimum distance from the dosing body 9) when the arm 8 is in the first position, i.e. when the dosing body 9 is in the loading position.

By changing the stroke of the plunger 3, i.e. the distance between the first and second position, the pressure value exerted by it changes.

In the embodiment described and illustrated herein, the device 1 comprises a box-like casing 30 provided with an upper cover 31.

At least the reservoir 2, the plunger 3, the compartment 5 for the compound, the dosing body 9 and the actuation means 8 are housed inside the box-like body 30.

In the embodiment described and illustrated herein, the mouthpiece 6 is rotatably mounted in the box-like casing 30.

In the resting position, the mouthpiece 6 is located inside the box-like casing 30, in particular below the cover 31, provided with a silicone protrusion 32 which prevents the intrusion of dust or objects inside the mouthpiece. In the operating position, the mouthpiece 6 extends at least partially from the box-like casing 30 so as to be accessible to the mouth of the patient.

In accordance with an embodiment not belonging to the invention, the reservoir 2 is selectively communicating with an environment outside the device 1 to contain a gaseous mixture. The plunger 3 defines a compressing means for compressing the gaseous mixture contained in the reservoir 2. Preferably, the gaseous mixture is air.

The compressed gaseous mixture is intended to act as a propellant for the compound.

In accordance with the embodiment not belonging to the invention, the gaseous mixture contained in the hollow cylindrical body 2 is compressed by pushing the plunger 3.

The device 1 comprises a one-way valve 4 configured to enable the selective communication of the reservoir 2 with the external environment.

In this embodiment not belonging to the invention, the dosing conduit 10 extends between a first end 10a and a second end 10b. In the loading position, the first end 10a is in communication with the compartment 5. In the administration position, the second end 10b is in communication with the reservoir 2 and the first end 10a communicates with the channel 7 of the mouthpiece 6.

The compressing means 3 is operatively connected to the arm 8 so as to act in compression of the gaseous mixture when the arm 8 moves towards the second position.

Thereby, with the sole movement of the arm 8 by the patient, the rotation of the dosing body 9 between the loading position and the administration position and the compression of the gaseous mixture is obtained.

There are two arms 8 which are arranged on opposite sides of the dosing body 9.

An administration cycle of the compound to the patient occurs as follows.

The arm 8 is brought into the first position, so that the dosing body 9 is in the loading position.

The dosing conduit 10 is thus in fluid communication with the compartment 5 and receives the predefined quantity of the compound.

During this loading step, the mouthpiece 6 is located inside the box-like body 30, not accessible to the patient.

Once the loading is completed, the arm 8 is moved from the first to the second position.

During the rotation of the arm 8 towards the second position, the dosing body 9 rotates between the loading position and the administration position while the gaseous mixture is simultaneously compressed by the compressing means 3.

When the arm 8 reaches the second position, the dosing conduit 10 communicates with the reservoir 2 upstream and with the channel 7 of the mouthpiece 6 downstream.

Meanwhile, the mouthpiece 6 has also changed configuration, moving to the operating position so that it partially extends outside the box-like body 30 to be accessible to the mouth of the patient.

The compressed gaseous mixture passes through the dosing conduit 10 to be dispensed from the channel 7 of the mouthpiece 6. Thereby, the gaseous mixture acts as a carrier, i.e. it carries the predetermined quantity of compound present in the dosing conduit 10, which thus enters the oral routes of the patient.

In accordance with the invention, the plunger 3 is operatively active on the reservoir 2 to generate a vacuum. In technical jargon, in this case the expression "making a vacuum" is used.

In particular, the plunger 3 generates a vacuum when it moves away from the dosing body 9, i.e. when it moves from the first position to the second position. This occurs when the dosing body 9 rotates towards the loading position.

Preferably, the device 1 comprises a conduit branch 16 originating from the reservoir 2. The dosing conduit 10 extends between a first end 10a and a second end 10b. In the loading position, the first end 10a is in communication with the compartment 5 and the second end 10b communicates directly with the reservoir 2. Thereby, as soon as the dosing body 9 reaches the loading position, the compound is sucked into the reservoir 2 through the dosing conduit 10, due to the vacuum generated by the plunger 3. The dosing conduit 10 and the reservoir 2 therefore define two "lungs" in series.

In the administration position, the first end 10a communicates with the channel 7 of the mouthpiece 6, while the second end 10b is in communication with the conduit branch 16 (and therefore indirectly with the reservoir 2).

The invention is preferably employed when the compound to be administered is a mixture. In this case, the compartment 5 is shaped to house a vial of compound. Usually, the vial is screwed into the compartment 5.

An administration cycle of the compound to the patient occurs as follows.

The arm 8 is brought into the first position, so that the dosing body 9 is in the loading position.

The dosing conduit 10 is thus in fluid communication with the compartment 5 on one side and with the reservoir 2 on the other. The plunger 3 generates a vacuum, so the reservoir 2 receives the predefined quantity of the compound in the form of a mixture through the dosing conduit 10.

During this loading step, the mouthpiece 6 is located inside the box-like body 30, not accessible to the patient.

Once the loading is completed, the arm 8 is moved from the first to the second position.

During the rotation of the arm 8 towards the second position, the dosing body 9 rotates between the loading position and the administration position while the compound mixture is simultaneously compressed by the plunger 3.

When the arm 8 reaches the second position, the dosing conduit 10 communicates with the reservoir 2 upstream and with the channel 7 of the mouthpiece 6 downstream.

Meanwhile, the mouthpiece 6 has also changed configuration, moving to the operating position so that it partially extends outside the box-like body 30 to be accessible to the mouth of the patient.

The compressed gaseous mixture passes through the branch 16, the dosing conduit 10 to be dispensed from the channel 7 of the mouthpiece 6.

In accordance with an alternative embodiment of the invention, the dosing conduit 10 has a three-way extension, thus comprising a first end 10a, a second end 10b and a third end 10c. In addition to what is described above for the second embodiment, the dosing conduit 10 is shaped so that in the loading position the third end 10c communicates with the external environment and in the administration position directly with the reservoir 2. Such an embodiment is preferable when the compound is in liquid form. In the loading position, the third end 10c integrates air, mixing it with the compound that is sucked by the vacuum of the reservoir 2.

Preferably, the mouthpiece 6 rotates around the axis X between the resting position and the operating position. In the preferred embodiment, the arm 8 and the mouthpiece 6 are mutually shaped so that in its movement towards the second position, the arm 8 forces the mouthpiece 6 to come to its operating position.

In an alternative embodiment, not illustrated, the mouthpiece 6 is manually configured in the operating position by the patient.

Preferably, the dosing body 9 has a substantially cylindrical shape. That is, the body 9 is a canister.

Preferably, the dosing body 9 comprises multiple dosing conduits 10 for simultaneously administering multiple drugs. Such dosing conduits 10 are independent of each other. That is, such dosing conduits 10 do not communicate with each other.

Preferably, the device 1 comprises a transformation means 11, 12 for transforming the rotational motion of the arm 8 into a translational motion of the compressing means 3.

In the embodiment described and illustrated herein, the transformation means comprises a pinion 11 integral with the arm 8 and a rack 12 integral with the compressing means 3. The rack 12 is slidingly coupled with the pinion 11. Thereby, the rotation of the pinion 11 (due to the arm 8) transforms into a translation of the rack 12 and therefore of the compressing means 3.

In the embodiment in which the compressing means 3 is a plunger, the plunger is connected to the rack 12.

As an alternative to the rack and pinion pair, it is possible to use any known mechanism or system that allows the transformation of a rotational motion into a translational motion.

In accordance with an embodiment, the device 1 comprises a mechanical or magnetic stop means 13 of the arm 8 in a third intermediate position between the first position and the second position. Thereby, the patient must exert a force greater than the resistant force exerted by the stop means 13 so that the arm 8 overcomes the third position and reaches the second position.

Once reaching the second position, the dosing conduit 10 enters in communication with the reservoir 2 upstream (indirectly through the branch 16) and with the channel 7 of the mouthpiece 6 downstream to dispense the predefined quantity of compound. In particular, in the embodiment not belonging to the invention, the compressed gaseous mixture of the reservoir 2 hits the predefined quantity of compound present in the dosing conduit 10 and carries it therewith in the channel 7 of the mouthpiece 6 and then in the oral routes of the patient.

In the invention, the compressed compound mixture in the reservoir 2 flows through the branch 16, the dosing conduit 10 and the channel 7 of the mouthpiece 6 to reach the oral routes of the patient.

In such an embodiment with the stop means 13, the triggering of the administration of the compound is given by the pushing effort of the patient on the arm 8.

Preferably, the stop means 13 is operatively active on the rack 12 to block the sliding thereof upon reaching a predefined position, corresponding to the third position of the arm 8. Preferably, one or more magnets are arranged inside the box-like casing 30 and one or more ferromagnetic portions are arranged on the rack 12, or vice versa.

Preferably, the device 1 comprises a counter of the number of administrations performed. The counter is configured to add one unit to a total value of administrations performed each time the arm 8 moves from the third position to the second position. In this embodiment, this step identifies the successful administration.

In accordance with another embodiment, the device 1 comprises a shutter 14 arranged inside the channel 7 of the mouthpiece 6. The shutter 14 is movable between a first position in which it allows the passage of fluid through the channel 7, and a second position, in which it prevents the passage of fluid in the channel 7. In this context, the first position of the shutter 14 is therefore associated with an open configuration of the channel 7, while the second position of the shutter 14 is associated with a closed configuration of the channel 7.

In particular, the shutter 14 is a cap.

In an embodiment, the shutter 14 is manually operated.

Preferably, the device 1 comprises a movement means for moving the shutter 14 between the first and the second position.

In an embodiment, the device 1 comprises a button 26 operatively connected to the movement means of the shutter 14. When the button 26 is pressed by the patient, the movement means configures the shutter 14 in the first open position.

In an embodiment, the movement means is shaped to activate in response to a sucking action of the patient on the mouthpiece 6.

In such an embodiment, the triggering of the administration of the compound is given by the sucking of the patient.

Preferably, the shutter 14 is a membrane with tension calibrated to open with a vacuum generated by the sucking of the patient. This allows to solve a synchronization problem of the patient's breathing with the triggering of the administration, which is critical for certain clinical profiles.

Preferably, the device 1 comprises a secondary channel 27 extending between an end communicating with the external environment and an end facing the dosing body 9. Preferably, the secondary channel 27 is made on the opposite side with respect to the mouthpiece 6. The device 1 comprises an air loading valve 28 arranged at the end communicating with the external environment to establish a selective communication therewith. In particular, the air loading valve 28 moves between a closed position and an open position.

Preferably, the button 26 is operatively connected to the air loading valve 28 and to the shutter 14 to synchronously configure them in the open or closed position.

Consider the embodiment in which the dosing conduit 10 is three-way.

In the embodiment not belonging to the invention, in which the gaseous mixture is used as a propellant, during the compound loading step the air loading valve 28 and the shutter 14 open. By acting on the arm 8 to bring the dosing body 9 into the loading position, the piston 3 moves towards the second position, i.e. the position of maximum distance from the dosing body 9. During this movement, the air is drawn back inside the reservoir 2. During this operation, the compound is brought from the compartment 5 to the dosing conduit 10.

Subsequently, the shutter 14 and the air loading valve 28 are closed and by acting on the arm 8, the dosing body 9 is brought towards the administration position. During this movement, the piston 3 moves towards the first position, i.e. the minimum distance position from the dosing body 9, putting pressure on the gaseous
mixture (air) contained in the reservoir 2. Upon reaching the second position, the patient can inhale and simultaneously act on the button 26 to trigger the administration. The air loading valve 28 and the shutter 14 open and the compressed air contained in the reservoir 2 flows through the dosing conduit 10 together with the ambient air entering through the secondary conduit 27. Such a flow hits and carries the compound present in the dosing channel 10 therewith and flows through the channel 7 into the oral routes of the patient.

In the invention , in which a vacuum is generated in the reservoir 2, during the compound loading step the air loading valve 28 and the shutter 14 are kept closed. By acting on the arm 8 to bring the dosing body 9 into the loading position, the piston 3 moves towards the second position, i.e. the position of maximum distance from the dosing body 9, generating the vacuum. During this operation, the compound is sucked thanks to the negative pressure created in the reservoir 2.

The rest of the operations are carried out in the same manner as described above.

Preferably, the presence of the shutter 14 inside the channel 7 allows to define a first segment and a second segment of the channel 7.

The shutter 14 is arranged between the first segment and the second segment so that in the first position it prevents the passage of fluid from the first segment to the second segment.

In particular, the first segment is closer to the axis X with respect to the second segment. The first segment communicates with the conduit 10 when the dosing body 9 is in the administration position.

Advantageously, the first segment has a transverse section of smaller dimensions than that of the second segment.

In accordance with a further embodiment, the device 1 comprises a tap 24 interposed between the reservoir 2 and the dosing body 9. When the dosing body 9 is in the administration position, the tap 24 puts the reservoir 2 and the dosing conduit 10 in communication. The tap 24 is configurable in an open position and in a closed position. The device 1 comprises a flow sensor 25 arranged in the channel 7 and configured to detect the sucking by the patient.

The device 1 comprises a motor M configured to open the tap 24 upon the detection of the sucking by the flow sensor 25.

In particular, the flow sensor 25 is configured to activate the motor M upon the detection of a greater flow rate with respect to a predetermined value. The motor M is configured for a modular opening of the shutter 14 as a function of the flow rate value detected by the flow sensor 25.

Alternatively, the shutter 14 can be present in place of the flow sensor 25. In an embodiment, the shutter 14 is configured to open when a predetermined pressure difference is reached either manually or after the button is pressed by the patient, as previously described.

In an alternative embodiment, the button 26 is present which is operatively connected to the shutter 14 and to the tap 24 to synchronously configure them in the closed or open position.

In accordance with an embodiment, the device 1 suitably comprises one or more air intakes 15 selectively communicating with the channel 7. The air intakes 15 are made so that the supplied air contributes to the opening of the shutter 14.

In particular, the air intakes 15 communicate with the first segment of the channel 7. The air intakes 15 are put in communication with the channel 7 when the patient sucks so that the additional air flow entering from the outside facilitates the opening of the shutter 14.

The device 1 comprises one or more closures for the air intakes 15. There can be a single closure for all the air intakes or one closure for each air intake 15. Each closure is movable between a closed position, in which it blocks the air intake15, and an open position, in which it allows the passage of air through the air intake 15.

The device 1 comprises a movement means for moving the closure between the two positions. The movement means is shaped to configure the closure in an open position in response to a sucking action of the patient on the mouthpiece 6.

Preferably, the opening and closing of the shutter 14 occur by common or synchronised movement means.

In the preferred embodiment, the predefined quantity of compound is contained inside a capsule. Therefore, the compartment 5 is shaped to receive a capsule.

Alternatively, the compartment 5 is shaped to receive another type of container for the compound, for example a vial. The type of container depends on the compound which is to be loaded in the device 1.

Preferably, the device 1 comprises a plurality of compartments 5. The compartments 5 are selectively positionable at a predefined zone in which they communicate with the dosing conduit 10 when the lung 9 is in the loading position. This meets the needs of polytherapy.

At such a predefined zone, the device 1 comprises a means for piercing the capsule.

In the embodiment disclosed and illustrated herein, the device 1 comprises a rotating drum 17 in which the compartments 5 are obtained. By rotating the drum 17, the desired compartment 5 is positioned at the predefined zone.

Since a different compound can be loaded into each compartment 5, it is of fundamental importance that the device 1 allows to precisely determine which compound is being administered.

In accordance with an embodiment, the compartments 5 each have a transverse section with a different shape.

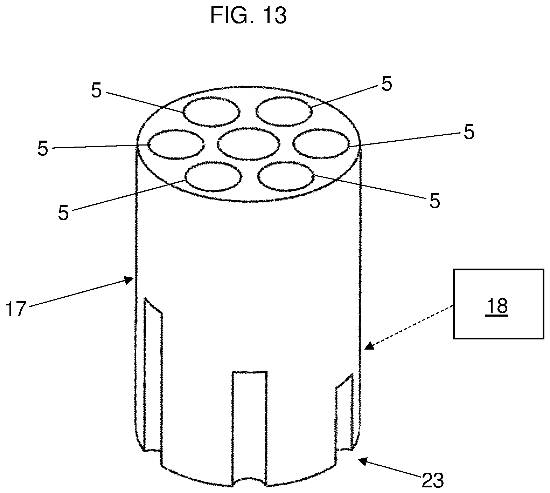

In accordance with an embodiment, the device 1 comprises a recognition system 23 for recognising the position of each compartment 5. The recognition system is of known type, for example mechanical, inductive, resistive, etc. In the exemplary embodiment illustrated in figure 13, each compartment 5 is associated with a notch or recess 23 of different dimensions (in this case length starting from the bottom of the drum 17). Alternatively, metal plates, electronic resistors, chips, etc. could be used.

Preferably, the device 1 comprises a control unit 18 configured to receive information on the position of each compartment 5 from the recognition system 23 and signal to the patient the position of the compartment 5 containing the compound capsule to be taken. Preferably, the control unit 18 is also configured to give instructions to the patient on how to bring the identified compartment 5 at the predefined zone.

Preferably, the device 1 is provided with a Bluetooth antenna 19 for connection with an application, for example on a mobile device, dedicated to supporting the patient in taking the drugs.

Preferably, the device 1 comprises a first compensation channel 20 and a second compensation channel 21, the latter obtained in the lung 9. The first compensation channel 20 communicates with an external environment, the second compensation channel 21 is shaped so as to put the first compensation channel 20 and the reservoir 2 in communication when the lung 9 is in the loading position. This helps supply the gaseous mixture from the outside towards the reservoir 2.

Preferably, the device 1 comprises an environment channel 22 arranged and shaped so that, when the lung 9 is in the administration position and the mouthpiece 6 is in the resting position, the first end 10a of the conduit is communicating therewith. This prevents the formation of vapours and the growth of bacteria during non-use of the device 1.

When the dosing conduit 10 is of the three-way type, in the loading position the third end 10c communicates with the environment channel 22

In the preferred embodiment, the box-like casing 30, as well as all the components of the device 1 are made of biodegradable material, for example PLA.

In the embodiment described and illustrated herein, the device 1 is portable. The box-like casing 30 contains all the cited components, in particular the body 9, the pinion 11, the rack 12, the compartment 5 and the reservoir 2.

The features of the device for administering a predefined quantity of a compound to a patient, according to the present invention, are clear from the description, as are the advantages.

In particular, the presence of the dosing conduit and its selective communication with the compartment housing the compound and with the reservoir allow to make an inhaler device which does not contain the compound already prepared therein. Conversely, the compound is loaded only if the patient intends to take it.

Furthermore, the connection between the arm, body and plunger allows in a single movement of the arm to compress the the compound mixture and to bring the conduit in communication with the reservoir. This allows to make a compact and safe portable device, as the patient only has to act on the arm with a simple movement.

Furthermore, the presence of the rotating drum with the recognition system for recognising the position of the compartments and the control unit allows to guide and assist the patient during the therapy, drastically reducing the possibility of error. This is particularly useful in the case of polytherapy, where the patient must take different drugs at specific times.

## Claims

1. A device (1) for administering a predefined quantity of a compound to a patient, comprising:
- a reservoir (2) of predefined volume;
- a plunger or piston (3) slidably mounted inside the reservoir (2);
- at least one compartment or housing (5) receiving a predefined quantity of the compound;
- a mouthpiece (6) configurable in at least one resting position, in which it is not accessible to a mouth of the patient, and an operating position, in which it is accessible to the mouth of the patient, said mouthpiece (6) comprising an internal channel (7) for expelling the compound;
- a dosing body (9) comprising at least one dosing conduit (10) of the compound;
- an actuation means (8) configured to move the dosing body (9) between a loading position, in which the dosing conduit (10) communicates with the compartment (5) to receive the predefined quantity of the compound, and an administration position, in which the dosing conduit (10) communicates with the reservoir (2) on one side and with the channel (7) of the mouthpiece (6) on the other, said plunger (3) being operatively connected to said actuation means (8),
**characterized in that** the plunger (3) is operatively active on the reservoir (2) to generate a vacuum when the dosing body (9) moves towards the loading position.

2. The device (1) according to claim 1, comprising a conduit branch (16) originating from the reservoir (2), said dosing conduit (10) extending between a first end (10a) and a second end (10b), said dosing conduit (10) being shaped so that in the loading position the first end (10a) communicates with the compartment (5) and the second end (10b) communicates directly with the reservoir (2), and in the administration position the first end (10a) communicates with the channel (7) and the second end (10b) communicates with the conduit branch (16).

3. The device (1) according to claim 2, wherein the dosing conduit (10) has a three-way extension and comprises a third end (10c), said dosing conduit (10) being shaped so that in the loading position the third end (10c) communicates with the external environment and in the administration position with the reservoir (2).

4. The device (1) according to any one of the preceding claims, wherein said actuation means (8) comprises at least one arm (8) rotatable about an axis (X) between a first position and a second position, said dosing body (9) being connected to said at least one arm (8) so as to rotate together therewith.

5. The device (1) according to claim 4, comprising a transformation means (11, 12) for transforming the rotational motion of said at least one arm (8) into a translational motion of the compressing means (3).

6. The device (1) according to claim 5, wherein said transformation means (11, 12) comprises a pinion (11) constrained to said at least one arm (8) and a rack (12) constrained to said compressing means (3), said rack (12) being slidably coupled with the pinion (11).

7. The device (1) according to any one of claims 4 to 6, further comprising:
- a shutter (14) arranged inside the channel (7) and movable between a first position, in which it allows the passage of fluid through the channel (7), and a second position, in which it prevents the passage of fluid through the channel (7);
- a movement means for moving the shutter (14) from the first position to the second position, said movement means being shaped to activate in response to a sucking action of the patient on the mouthpiece (6).

8. The device (1) according to claim 7, wherein the channel (7) comprises a first segment and a second segment, said first segment having a transverse section of smaller dimensions than that of the second segment, said shutter (14) being arranged so as to prevent, in the second position, the passage of fluid from the first segment to the second segment.

9. The device (1) according to claim 8, further comprising:
- one or more air intakes (15) selectively communicating with the channel (7);
- one closure for each air intake (15) which is movable between a closed position, in which it occludes the air intake (15), and an open position, in which it allows the passage of air through the air intake (15);
- a movement means for moving said closure between the two positions, said movement means being shaped to configure the closure in an open position in response to the sucking of the patient.

10. The device (1) according to any one of the preceding claims, wherein said at least one compartment (5) is shaped to receive a capsule containing said predefined quantity of the compound.

11. The device (1) according to claim 10, comprising a plurality of compartments (5) selectively positionable at a predefined zone in which they communicate with the conduit (10) in the loading position of the dosing body (9).

12. The device (1) according to claim 11, further comprising:
- a recognition system (23) for recognising the position of each of the compartments (5);
- a control unit (18) configured to receive information on the position of each compartment (5) from said recognition system (23) and provide instructions or information to the patient about a specific compartment (5) to be selected.

## Patentansprüche

1. Vorrichtung (1) zur Verabreichung einer vorbestimmten Menge einer Verbindung an einen Patienten, umfassend:
- ein Reservoir (2) eines vorbestimmten Volumens;
- eine Kolbenstange oder einen Kolben (3), die bzw. der verschiebbar innerhalb des Reservoirs (2) montiert ist;
- mindestens eine Unterteilung oder Einhausung (5), die eine vorbestimmte Menge der Verbindung aufnimmt;
- ein Mundstück (6), das in mindestens einer Ruheposition, in der es nicht für den Mund des Patienten zugänglich ist, und einer Betriebsposition, in der es für den Mund des Patienten zugänglich ist, auslegbar ist, wobei das Mundstück (6) einen internen Kanal (7) zum Ausstoßen der Verbindung umfasst;
- einen Dosierkörper (9), umfassend mindestens eine Dosierleitung (10) der Verbindung;
- Betätigungsmittel (8), die ausgelegt sind, um den Dosierkörper (9) zwischen einer Ladeposition, in der die Dosierleitung (10) mit der Unterteilung (5) kommuniziert, um die vorbestimmte Menge der Verbindung aufzunehmen, und einer Verabreichungsposition, in der die Dosierleitung (10) mit dem Reservoir (2) auf einer Seite und mit dem Kanal (7) des Mundstücks (6) auf der anderen kommuniziert, zu bewegen, wobei die Kolbenstange (3) betriebswirksam mit den Betätigungsmitteln (8) verbunden ist,
**dadurch gekennzeichnet, dass** die Kolbenstange (3) betriebswirksam auf das Reservoir (2) wirkt, um ein Vakuum zu erzeugen, wenn sich der Dosierkörper (9) hinführend zur Ladeposition bewegt.

2. Vorrichtung (1) nach Anspruch 1, umfassend einen Leitungsabzweig (16), der von dem Reservoir (2) abgeht, wobei sich die Dosierleitung (10) zwischen einem ersten Ende (10a) und einem zweiten Ende (10b) erstreckt, wobei die Dosierleitung (10) so ausgeformt ist, dass das erste Ende (10a) in der Ladeposition mit der Unterteilung (5) kommuniziert und das zweite Ende (10b) direkt mit dem Reservoir (2) kommuniziert und das erste Ende (10a) in der Verabreichungsposition mit dem Kanal (7) kommuniziert und das zweite Ende (10b) mit dem Leitungsabzweig (16) kommuniziert.

3. Vorrichtung (1) nach Anspruch 2, wobei die Dosierleitung (10) eine Dreiwege-Ausdehnung aufweist und ein drittes Ende (10c) umfasst, wobei die Dosierleitung (10) so ausgeformt ist, dass das dritte Ende (10c) in der Ladeposition mit der äußeren Umgebung und in der Verabreichungsposition mit dem Reservoir (2) kommuniziert.

4. Vorrichtung (1) nach einem der vorhergehenden Ansprüche, wobei die Betätigungsmittel (8) mindestens einen Arm (8) umfassen, der um eine Achse (X) zwischen einer ersten Position und einer zweiten Position drehbar ist, wobei der Dosierkörper (9) mit dem mindestens einen Arm (8) so verbunden ist, dass er sich mit diesem zusammen dreht.

5. Vorrichtung (1) nach Anspruch 4, umfassend Umwandlungsmittel (11, 12), um die Drehbewegung des mindestens einen Arms (8) in eine translatorische Bewegung der Kompressionsmittel (3) umzuwandeln.

6. Vorrichtung (1) nach Anspruch 5, wobei die Umwandlungsmittel (11, 12) ein Ritzel (11) umfassen, das an dem mindestens einen Arm (8) befestigt ist, und ein Gestell (12), das an den Kompressionsmitteln (3) befestigt ist, wobei das Gestell (12) verschiebbar mit dem Ritzel (11) gekuppelt ist.

7. Vorrichtung (1) nach einem der Ansprüche 4 bis 6, ferner umfassend:
- einen Schließer (14), der innerhalb des Kanals (7) angeordnet und zwischen einer ersten Position, in der er das Strömen von Fluid durch den Kanal (7) erlaubt, und einer zweiten Position, in der er das Strömen von Fluid durch den Kanal (7) verhindert, bewegbar ist;
- Bewegungsmittel zum Bewegen des Schließers (14) von der ersten Position in die zweite Position, wobei die Bewegungsmittel ausgeformt sind, um als Reaktion auf eine Saugwirkung des Patienten auf das Mundstück (6) aktiviert zu werden.

8. Vorrichtung (1) nach Anspruch 7, wobei der Kanal (7) ein erstes Segment und ein zweites Segment umfasst, wobei das erste Segment einen Querschnitt kleinerer Abmessungen als das zweite Segment aufweist, wobei der Schließer (14) so angeordnet ist, dass er in der zweiten Position das Strömen von Fluid von dem ersten Segment zu dem zweiten Segment verhindert.

9. Vorrichtung (1) nach Anspruch 8, ferner umfassend:
- einen oder mehrere Lufteinlässe (15), die wahlweise mit dem Kanal (7) kommunizieren;
- einen Verschluss für jeden Lufteinlass (15), der zwischen einer geschlossenen Position, in der er den Lufteinlass (15) versperrt, und einer offenen Position, in der er das Strömen von Luft durch den Lufteinlass (15) erlaubt, bewegbar ist;
- Bewegungsmittel zum Bewegen des Verschlusses zwischen den beiden Positionen, wobei die Bewegungsmittel ausgeformt sind, um den Verschluss als Reaktion auf das Saugen des Patienten in einer offenen Position auszulegen.

10. Vorrichtung (1) nach einem der vorhergehenden Ansprüche, wobei die mindestens eine Unterteilung (5) ausgeformt ist, um eine Kapsel aufzunehmen, die die vorbestimmte Menge der Verbindung enthält.

11. Vorrichtung (1) nach Anspruch 10, umfassend eine Vielzahl von Unterteilungen (5), die wahlweise an einer vorbestimmten Zone positionierbar sind, in der sie mit der Leitung (10) in der Ladeposition des Dosierkörpers (9) kommunizieren.

12. Vorrichtung (1) nach Anspruch 11, ferner umfassend:
- ein Erkennungssystem (23) zum Erkennen der Position einer jeden der Unterteilungen (5);
- eine Steuereinheit (18), die ausgelegt ist, um Informationen über die Position einer jeden Unterteilung (5) von dem Erkennungssystem (23) zu empfangen und dem Patienten Anweisungen oder Informationen über eine spezifische auszuwählende Unterteilung (5) bereitzustellen.

## Revendications

1. Dispositif (1) pour administrer une quantité prédéfinie d'un composé à un patient, comprenant :
- un réservoir (2) de volume prédéfini ;
- un plongeur ou piston (3) monté de manière coulissante à l'intérieur du réservoir (2) ;
- au moins un compartiment ou logement (5) recevant une quantité prédéfinie du composé ;
- un embout buccal (6) configurable dans au moins une position de repos, dans laquelle il n'est pas accessible à la bouche du patient, et une position de fonctionnement, dans laquelle il est accessible à la bouche du patient, ledit embout buccal (6) comprenant un canal interne (7) pour expulser le composé ;
- un corps de dosage (9) comprenant au moins un conduit de dosage (10) du composé ;
- des moyens d'actionnement (8) configurés pour déplacer le corps de dosage (9) entre une position de chargement, dans laquelle le conduit de dosage (10) communique avec le compartiment (5) pour recevoir la quantité prédéfinie du composé, et une position d'administration, dans laquelle le conduit de dosage (10) communique avec le réservoir (2) d'un côté et avec le canal (7) de l'embout buccal (6) de l'autre, ledit plongeur (3) étant relié de manière opérationnelle auxdits moyens d'actionnement (8),
**caractérisé en ce que** le plongeur (3) est actif de manière opérationnelle sur le réservoir (2) pour générer un vide lorsque le corps de dosage (9) se déplace vers la position de chargement.

2. Dispositif (1) selon la revendication 1, comprenant une branche de conduit (16) provenant du réservoir (2), ledit conduit de dosage (10) s'étendant entre une première extrémité (10a) et une deuxième extrémité (10b), ledit conduit de dosage (10) étant formé de sorte que dans la position de chargement, la première extrémité (10a) communique avec le compartiment (5) et la deuxième extrémité (10b) communique directement avec le réservoir (2), et dans la position d'administration, la première extrémité (10a) communique avec le canal (7) et la deuxième extrémité (10b) communique avec la branche de conduit (16).

3. Dispositif (1) selon la revendication 2, dans lequel le conduit de dosage (10) a une extension à trois voies et comprend une troisième extrémité (10c), ledit conduit de dosage (10) étant formé de sorte que dans la position de chargement, la troisième extrémité (10c) communique avec l'environnement extérieur et dans la position d'administration avec le réservoir (2).

4. Dispositif (1) selon l'une quelconque des revendications précédentes, dans lequel lesdits moyens d'actionnement (8) comprennent au moins un bras (8) pouvant tourner autour d'un axe (X) entre une première position et une seconde position, ledit corps de dosage (9) étant relié audit au moins un bras (8) de manière à tourner avec celui-ci.

5. Dispositif (1) selon la revendication 4, comprenant des moyens de transformation (11, 12) pour transformer le mouvement de rotation dudit au moins un bras (8) en un mouvement de translation des moyens de compression (3).

6. Dispositif (1) selon la revendication 5, dans lequel lesdits moyens de transformation (11, 12) comprennent un pignon (11) contraint audit au moins un bras (8) et une crémaillère (12) contrainte auxdits moyens de compression (3), ladite crémaillère (12) étant couplée de manière coulissante au pignon (11).

7. Dispositif (1) selon l'une quelconque des revendications 4 à 6, comprenant de plus :
- un obturateur (14) disposé à l'intérieur du canal (7) et mobile entre une première position, dans laquelle il permet le passage de fluide à travers le canal (7), et une seconde position, dans laquelle il empêche le passage de fluide à travers le canal (7) ;
- des moyens de déplacement pour déplacer l'obturateur (14) de la première position à la seconde position, lesdits moyens de déplacement étant formés pour s'activer en réponse à une action de succion du patient sur l'embout buccal (6).

8. Dispositif (1) selon la revendication 7, dans lequel le canal (7) comprend un premier segment et un second segment, ledit premier segment ayant une section transversale de dimensions inférieures à celle du second segment, ledit obturateur (14) étant disposé de manière à empêcher, dans la seconde position, le passage de fluide du premier segment au second segment.

9. Dispositif (1) selon la revendication 8, comprenant de plus :
- une ou plusieurs entrées d'air (15) communiquant de manière sélective avec le canal (7) ;
- une fermeture pour chaque entrée d'air (15) qui est mobile entre une position fermée, dans laquelle elle obture l'entrée d'air (15), et une position ouverte, dans laquelle elle permet le passage de l'air à travers l'entrée d'air (15) ;
- des moyens de déplacement pour déplacer ladite fermeture entre les deux positions, lesdits moyens de déplacement étant formés pour configurer la fermeture dans une position ouverte en réponse à la succion du patient.

10. Dispositif (1) selon l'une quelconque des revendications précédentes, dans lequel ledit au moins un compartiment (5) est formé pour recevoir une capsule contenant ladite quantité prédéfinie du composé.

11. Dispositif (1) selon la revendication 10, comprenant une pluralité de compartiments (5) positionnables de manière sélective dans une zone prédéfinie dans laquelle ils communiquent avec le conduit (10) dans la position de chargement du corps de dosage (9).

12. Dispositif (1) selon la revendication 11, comprenant de plus :
- un système de reconnaissance (23) pour reconnaître la position de chacun des compartiments (5) ;
- une unité de contrôle (18) configurée pour recevoir des informations sur la position de chaque compartiment (5) à partir dudit système de reconnaissance (23) et fournir des instructions ou des informations au patient concernant un compartiment spécifique (5) à sélectionner.
